# EUROPÄISCHE PATENTSCHRIFT

(11) **EP 0 692 479 B1**
(45) Veröffentlichungstag und Bekanntmachung des Hinweises auf die Patenterteilung: **19.11.1997**
(21) Anmeldenummer: 95110210.2
(22) Anmeldetag: 30.06.1995
(51) Int. Cl.: C07D 291/06, C07B 39/00

(54) **1,2,3-Oxathiazin-4(3F)-on-2,2-dioxide, ein Verfahren zu ihrer Herstellung und ihre Verwendung als elektrophile Fluorierungsmittel**
1,2,3-Oxathiazin-4(3F)-one-2,2-dioxides, process for their preparation and their use as electrophilic fluorination agents
1,2,3-Oxythiazin-4(3F)-one-2,2-dioxydes, procédé pour leur préparation et leur utilisation comme agents de fluoration électrophiles

(30) Priorität: 13.07.1994 DE 4425407
(43) Veröffentlichungstag der Anmeldung: 17.01.1996
(73) Patentinhaber: HOECHST AKTIENGESELLSCHAFT, 65929 Frankfurt am Main (DE)
(72) Erfinder: Cabrera, Ivan, Dr., D-63303 Dreieichenhain (DE); Appel, Wolfgang, Dr., D-65779 Kelkheim (DE)

(56) Entgegenhaltungen:
- EP-A- 0 211 578
- TETRAHEDRON, Bd. 47, Nr. 35, 26.August 1991 OXFORD GB, Seiten 7447-7458, A.A. GAKH ET AL. 'N-Fluorination with cesium fluoroxysulfate'

## Beschreibung

Die Erfindung betrifft 1,2,3-Oxathiazin-4(3F)-on-2,2-dioxide, ein Verfahren zu ihrer Herstellung und ihre Verwendung als elektrophile Fluorierungsmittel.

Viele neue Agro- und Pharmawirkstoffe enthalten F-Atome an strategischen Positionen. Ein Grund hierfür ist die Tatsache, daß der Ersatz von Wasserstoff durch Fluor (isosterische Substitution) oder von Hydroxylgruppen durch Fluor (isopolare Substitution) sehr oft zu einer Verbesserung der Wirksamkeit führt. Die selektive Einführung von Fluor in organische Moleküle ist deshalb eine sehr wichtige Aufgabe in der modernen Chemie geworden. Obwohl durch die Einführung von Diethylamino-Schwefeltrifluorid und anderer Reagenzien dieser Verbindungsklasse ein Durchbruch auf dem Gebiet der nukleophilen Fluorierung erreicht wurde, besteht weiterer Bedarf an sicheren, milden und effizienten elektrophilen Fluorierungsmitteln. Die meisten solcher elektrophilen Reagenzien, wie z. B. Perchlorylfluorid, Trifluormethylhypofluorid, CsSO₄F usw. sind toxische und sehr aggressive Chemikalien, mit denen nicht selten Explosionen beobachtet wurden. Darüberhinaus ist die Lagerstabilität solcher Materialien sehr begrenzt. "F^{⊕}"-Reagenzien auf der Basis von N-F haltigen Verbindungen wurden sehr intensiv untersucht, da einige dieser Materialien sich als gut isolierbar, lagerstabile und effiziente Fluorierungsmitteln bewährt haben. Erste Versuche in dieser Richtung wurden mit Perfluor-N-Fluorpiperidin (A) (J. Chem. Soc. Perkin Trans. I 1988, 2805) durchgeführt. Aufgrund der aufwendigen Synthese (Ausbeute max. 13 %) und der Nebenreaktion während der Fluorierung ist diese Verbindung für praktische Zwecke jedoch nicht von Interesse. Andere bekannte N-F Fluorierungsmittel sind N-Fluorpyridin-2-(1H)one (B) (J. Org. Chem. 1983, 43, 761), N-Fluorsulfonamide (C) (U.S. Pat. 4,479,901, 4,828,764, DE 36 23 184 A); Camphor-N-Fluor-Sultam (D) (Tetrahedron Lett. 1988, 29, 6087); N-Fluorquinicludinium Salze (E) (J. Chem. Soc. Perkin Trans I, 1988, 2805); N-Fluorpyridinium Salze (F) (J. Am. Chem. Soc. 1990, 112, 8563); N-Fluor-N-Perfluormethyl Sulfonamides (G) (U.S. Pat. 4,828,764, U.S. Pat. 5,227,493) und N-Fluor-N-chlormethyl-triethylendiamin-bis(tetrafluorborat) (F-Teda (H)) (U.S. Pat. 5,086,178).

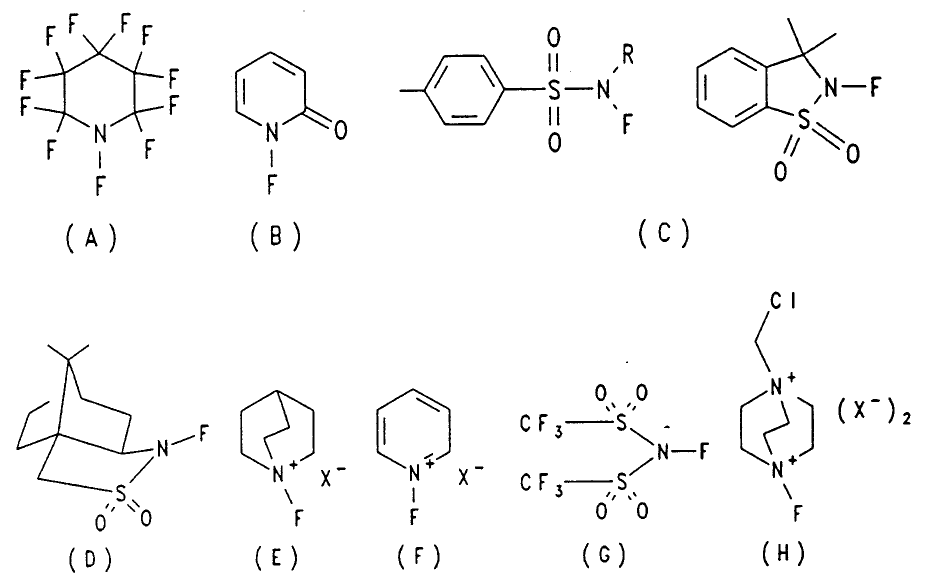

Die Verbindung (B) ist nicht lagerstabil. Das Reagenz (G), das die stärkste bekannte NF-Verbindung darstellt, erfordert eine sehr aufwendige Synthese für seine Herstellung. Die Verbindungen (C), (F) und (H) sind kommerziell erhältlich. Die käuflichen N-Flurosulfonamide haben allerdings den Nachteil, daß aufgrund des Wasserstoffatoms an der α-Stelle des N-Alkylrests eine HF-Eliminierung sehr leicht als Nebenreaktion auftreten kann. Mit N-Alkylresten, die keine Wasserstoffatome in der α-Stelle besitzen, z. B. einer t-Butylgruppe, ist die Ausbeute bei der Herstellung der NF-Verbindung sehr gering. Obwohl die geladenen Systeme (H), (F), sehr effiziente Fluorierungsmittel sind, ist ein entscheidender Nachteil dieser Systeme ihre begrenzte Löslichkeit in den gängigen organischen Lösungsmitteln. F-Teda (H) hat zusätzlich den Nachteil, daß bei diesem quaternären Ammoniumsalz oft eine Hofmann-Eliminierung stattfindet. Dies ist besonders problematisch bei der Fluorierung von starken Carbanionen.

In Tetrahedron Vol. 47, No. 35, 1991 Seiten 7447 bis 7458 ist die Verwendung von Cäsiumfluroxysulfat als elektrophiles Fluorierungsmittel für die Herstellung von N-Fluorverbindungen beschrieben. So wird durch Umsetzung von Saccharin mit Cäsiumfluoroxysulfat N-Fluorsaccharin erhalten, dem die Autoren Bedeutung als elektrophiles Fluorierungsmittel bemessen. Eine Herstellung von N-Fluorsaccharin in größerem Maßstab durch Fluorierung von Sacharin mit elementaren Fluor schlug jedoch fehl, bei dieser Reaktion trat Ringöffnung des Heterocyclus auf.

Es bestand daher ein großer Bedarf nach einem elektrophilen Fluorierungsmittel, das die beschriebenen Nachteile nicht aufweist, aus gut zugänglichen Edukten einfach herzustellen ist und hohe Lagerstabilität besitzt.

Diese Aufgabe wird gelöst durch Verbindungen der allgemeinen Formel (I) 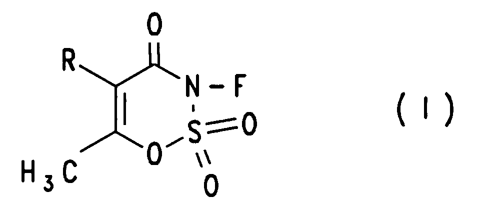 worin R Wasserstoff oder (C₁-C₄)-Alkyl bedeutet.

Von Interesse sind die Verbindungen der Formel (I) worin R gleich Wasserstoff oder Methyl ist.

Abhängig vom Lösungsmittel ist es auch möglich, daß die Verbindungen der Formel (I) im tautomeren Gleichgewicht mit den Verbindungen der Formel (I') stehen:

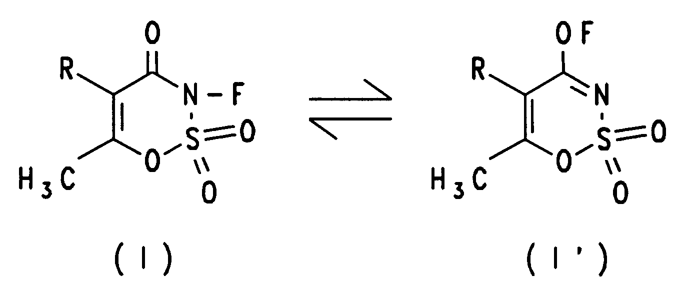

Die Erfindung betrifft ferner ein Verfahren zur Herstellung der Verbindungen der Formel (I). Es ist dadurch gekennzeichnet, daß man Verbindungen der Formel (II), worin R die oben erwähnte Bedeutung besitzt, und X für Wasserstoff oder ein Alkalimetall steht, in Gegenwart eines inerten Lösungsmittels und gegebenenfalls eines Alkalimetallfluorids bei tiefen Temperaturen mit elementarem Fluor umsetzt.

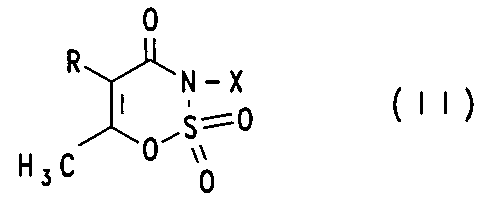

Die Verbindungen (II) sind großtechnische Produkte (R = H) oder auf literaturbekannte Weise herzustellen.

Gute Ergebnisse werden z. B. erhalten, wenn X für Wasserstoff, Natrium oder Kalium steht.

Als Alkalimetallfluoride haben sich Natrium- oder Kaliumfluorid, insbesondere Natriumfluorid bewährt.

Das Fluorierungsmittel Fluor wird vorteilhaft im Gemisch mit Inertgas wie Stickstoff, SF₆, CF₄ oder Edelgasen wie Helium, Neon, Argon, Krypton eingesetzt. Bevorzugtes Inertgas ist Stickstoff. Für die Fluorierung können Fluor/Inertgas-Gemische eingesetzt werden, die bis zu 30 Vol.-% Fluor enthalten.

Es hat sich in vielen Fällen bewährt, die Fluorierung mit einem N₂/F₂-Gemisch vorzunehmen, das zwischen 1 und 15 Vol.-%, insbesondere 2 und 10 Vol.-%, bevorzugt 3 bis 6 Vol.-% F₂ enthält.

Als Lösungsmittel sind z. B. Halogenkohlenwasserstoffe wie Dichlormethan, Chloroform, Tetrachlorkohlenstoff, Fluortrichlormethan, Trifluor-Trichloräthan, Tetrafluor-Dichloräthan oder Nitrile, insbesondere Acetonitril geeignet.

Die Temperatur bei der die Umsetzung durchgeführt wird, kann in weiten Bereichen variiert werden und liegt insbesondere im Bereich von + 10 bis -80°C. Die Wahl der Temperatur hängt im Einzelfall von der Wahl der Reaktionsbedingungen wie Fluorkonzentration, Zusammensetzung des Lösungsmittelgemisches etc. ab.
Die Fluorierung wird vorteilhaft bei Temperaturen von -80 bis -20°C, insbesondere -60 bis -30°C, bevorzugt -50 bis -35°C durchgeführt.

Die Erfindung betrifft weiterhin die Verwendung der Verbindungen der Formel (I) zur Fluorierung von Verbindungen die offenen oder versteckten Carbanion-Charakter besitzen, wie z. B. 1,3-Dicarbonylverbindungen. Diese Verbindungen können in sehr hohen Ausbeuten zu den Fluorverbindungen umgesetzt werden.

Die Verbindungen der Formel (I) sind in der Lage Iod zu erzeugen, wenn man sie mit Natriumiodidlösung behandelt. Die Konzentration der Verbindungen kann deshalb mittels Titration festgestellt werden.

### Beispiel 1

### 5,6-Dimethyl-1,2,3-oxathiazin-4(3F)-on-2,2-dioxid

In der vollständig trockenen (ausgeflammten) unter N₂ stehenden Fluorierungsapparatur werden 1,08 g (5,4 mmol) 5,6-Dimethyl-1,2,3-oxathiazin-4(3H)-on-2,2-dioxid Natriumsalz in 120 ml mit CaH₂ getrocknetem Acetonitril (Chromasolv R für HPLC) suspendiert, mit 0,22 g NaF (5,4 mmol) versetzt un bei -40°C mit einer 5 % (v/v) F₂ in N₂-Mischung fluoriert. Anschließend wird 0,5 h bei -40°C und 1 h bei Raumtemperatur mit N₂ gespült. Das Gemisch wird filtriert und das Filtrat an der Ölpumpe schnell abdestilliert. Das gelbe Öl wird in getrocknetem Ether aufgenommen, klar filtriert und am Rotationsverdampfer eingeengt. Nach dem Trocknen an der Ölpumpe erhält man 0,55 g Produkt (hellgelbes Öl).
¹H-NMR (300-MHz, CDCl₃): δ(ppm) 2.02 (Quintett, CH₃-C-CO); 2,30 (Quartett, CH₃-C-O). ¹⁹F-NMR (94,2 MHz, CH₃CN): δ(ppm, Standard CFCl₃) - 78 (b,NF).

### Beispiel 2

### 6-Methyl-1,2,3-oxathiazin-4(3F)-on-2,2-dioxid

Fluorierung wie oben beschrieben von einer Mischung aus 1,38 g Acesulfam (6-Methyl-1,2,3-oxathiazin-4(3H)-on-2,2-dioxid) und 1,78 g NaF in 120 ml Acetonitril gab 0,92 g eines gelben Öls.
¹H-NMR (100 MHz, CDCld₃): δ (ppm) 2,18 (d, CH₃-); 5,81 (m, 1H). ¹⁹F-NMR (94,2 MHz, CH₃CN): δ (ppm, Standard CFCl₃): -78,8 (b,NF).

Folgende Beispiele sollen die Verwendung der oben beschriebenen Öle illustrieren:

### Beispiel 3

### Cyclopentanon-2-fluor-2-carbonsäureethylester

Eine Lösung von 207 mg (1,3 mmol) Cyclopentanon-2-carbonsäureethylester in 60 ml getrocknetem THF wurde zu einer Suspension von 42 mg NaH (80 % in Öl / 10 ml THF) bei 0°C unter Ar zugegeben. Die Mischung wurde 0,5 h bei 0°C und 1 h bei Raumtemperatur gerührt bevor das Öl von Beispiel 2 (0,5 g) zugegeben wurde. Die Reaktionsmischung wurde 4 h bei Raumtemperatur gerührt. Das Lösungsmittel wurde am Rotationsverdampfer abgezogen. Der Rückstand wurde in Ether aufgenommen, klar filtriert und das Filtrat mit Wasser, gesät. NaHCO₃ Lösung und Wasser ausgeschüttet. Die Etherphase wurde dann mit Na₂SO₄ getrocknet, abfiltriert und das Lösungsmittel unter Vakuum entfernt. Ausbeute 152 mg (67 % d.Th.).

### Beispiel 4

### 2-Fluor-2-phenylmalonsäurediethylester

Eine Lösung von 284 mg (1,2 mmol) 2-Phenylmalonsäurediethylester in 50 ml getrocknetem THF wurde zu einer Suspension von 36 mg NaH (80 % in Öl / 10 ml THF) bei 0°C unter Ar zugegeben. Die Mischung wurde 0,5 h bei 0°C und 1 h bei Raumtemperatur gerührt bevor das Öl von Beispiel 1 (0,3 g) zugegeben wurde. Die Reaktionsmischung wurde 4 h bei Raumtemperatur gerührt. Das Lösungsmittel wurde am Rotationsverdampfer abgezogen. Der Rückstand wurde in Ether aufgenommen, klar filtriert und das Filtrat mit Wasser, gesät. NaHCO₃ Lösung und Wasser ausgeschüttelt. Die Etherphase wurde dann mit Na₂SO₄ getrocknet, abfiltriert und das Lösungsmittel unter Vakuum entfernt. Ausbeute 250 mg (82 % d.Th.).

## Patentansprüche

1. Verbindungen der allgemeinen Formel (I) 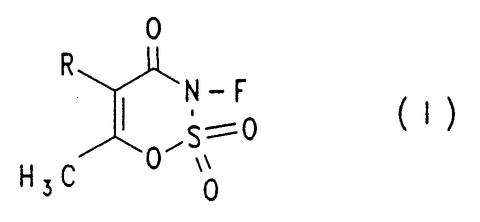 worin R Wasserstoff oder (C₁-C₄)-Alkyl bedeutet.

2. Verbindungen der allgemeinen Formel (I), worin R Wasserstoff oder Methyl bedeuten.

3. Verfahren zur Herstellung der Verbindung nach Anspruch 1 oder 2, dadurch gekennzeichnet, daß man Verbindungen der allgemeinen Formel (II) 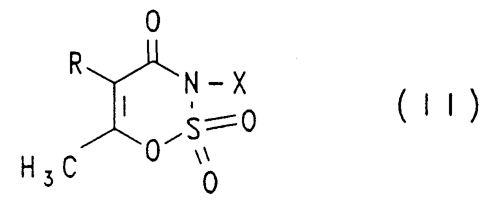 worin R die angegebene Bedeutung besitzt und X für Wasserstoff oder ein Alkalimetall steht, in Gegenwart eines inerten Lösungsmittels und gegebenenfalls eines Alkalimetallfluorids bei niedrigen Temperaturen mit elementarem Fluor umsetzt.

4. Verfahren nach Anspruch 3, dadurch gekennzeichnet, daß X für Wasserstoff, Natrium oder Kalium steht.

5. Verfahren nach Anspruch 3 oder 4, dadurch gekennzeichnet, daß als Alkalimetallfluorid Natrium- oder Kaliumfluorid, insbesondere Natriumfluorid eingesetzt wird.

6. Verfahren nach mindestens einem der Ansprüche 3 bis 5, dadurch gekennzeichnet, daß das Fluorierungsmittel Fluor im Gemisch mit Inertgasen wie Stickstoff, SF₆, CF₄ oder Edelgasen wie Helium, Neon, Argon, Krypton, insbesondere Stickstoff eingesetzt wird.

7. Verfahren nach mindestens einem der Ansprüche 3 bis 6, dadurch gekennzeichnet, daß die Fluorierung mit einem N₂/F₂-Gemisch durchgeführt wird, das zwischen 1 und 15 Vol.-%, insbesondere 2 und 10 Vol.- %, bevorzugt 3 bis 6 Vol.-% F₂ enthält.

8. Verfahren nach mindestens einem der Ansprüche 3 bis 7, dadurch gekennzeichnet, daß als Lösungsmittel Halogenkohlenwasserstoffe, insbesondere Dichlormethan, Chloroform, Tetrachlorkohlenstoff, Fluortrichlormethan, Trifluor-Trichloräthan, Tetrafluor-Dichloräthan oder Nitrile, insbesondere Acetonitril verwendet werden.

9. Verfahren nach mindestens einem der Ansprüche 3 bis 8, dadurch gekennzeichnet, daß die Fluorierung bei Temperaturen von -80 bis + 10°C, insbesondere -80 bis -20°C, bevorzugt -60 bis -30°C, besonders bevorzugt -50 bis -35°C durchgeführt wird.

10. Verwendung von Verbindungen der Formel (I) zur Fluorierung von Verbindungen die offenen oder versteckten Carbanioncharakter besitzen.

11. Verwendung nach Anspruch 10, dadurch gekennzeichnet, daß als Verbindungen mit Carbanioncharakter 1,3-Dicarbonylverbindungen eingesetzt werden.

## Claims

1. A compound of the formula (I) 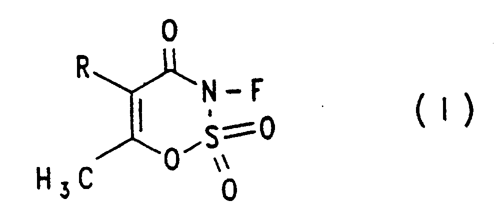 in which R is hydrogen or (C₁-C₄)-alkyl.

2. A compound of the formula (I) in which R is hydrogen or methyl.

3. A process for the preparation of a compound as claimed in claim 1 or 2, which comprises reacting a compound of the formula (II) 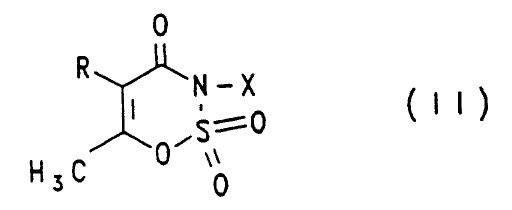 in which R is as defined and X is hydrogen or an alkali metal in the presence of an inert solvent and, if desired, of an alkali metal fluoride with elemental fluorine at low temperatures.

4. The process as claimed in claim 3, wherein X is hydrogen, sodium or potassium.

5. The process as claimed in claim 3 or 4, wherein sodium fluoride or potassium fluoride, especially sodium fluoride, is employed as alkali metal fluoride.

6. The process as claimed in at least one of claims 3 to 5, wherein the fluorinating agent, fluorine, is employed in a mixture with an inert gas such as nitrogen, SF₆ or CF₄ or a noble gas such as helium, neon, argon or krypton, especially nitrogen.

7. The process as claimed in at least one of claims 3 to 6, wherein the fluorination is carried out using an N₂/F₂ mixture which contains between 1 and 15% by volume, in particular between 2 and 10% by volume, preferably from 3 to 6% by volume, of F₂.

8. The process as claimed in at least one of claims 3 to 7, wherein the solvent used is a halogenated hydrocarbon, especially dichloromethane, chloroform, carbon tetrachloride, fluorotrichloromethane, trifluorotrichloroethane or tetrafluorodichloroethane or a nitrile, especially acetonitrile.

9. The process as claimed in at least one of claims 3 to 8, wherein the fluorination is carried out at temperatures of from -80 to +10°C, in particular from -80 to -20°C, preferably from -60 to -30°C and with particular preference from -50 to -35°C.

10. The use of a compound of the formula (I) for the fluorination of compounds possessing an open or concealed carbanion character.

11. The use as claimed in claim 10, wherein the compounds having a carbanion character which are employed are 1,3-dicarbonyl compounds.

## Revendications

1. Composés de formule générale (I) 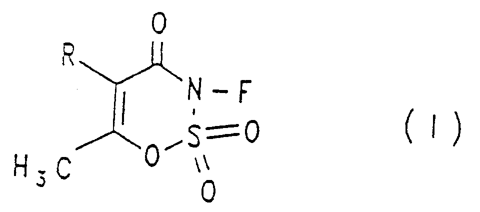 dans laquelle R est un atome d'hydrogène ou alkyle en C₁-C₄.

2. Composés de formule générale (I) dans laquelle R est l'hydrogène ou méthyle.

3. Procédé pour préparer le composé selon la revendication 1 ou 2, caractérisé en ce que l'on fait réagir avec du fluor élémentaire des composés de formule générale (II) 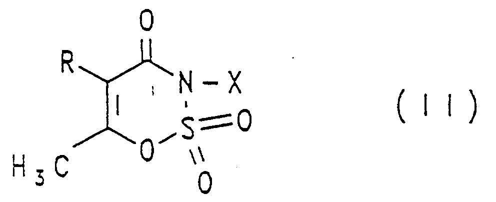 dans laquelle R a la signification donnée ci-dessus et X est un atome d'hydrogène ou un métal alcalin, en présence de solvant inerte et éventuellement d'un fluorure de métal alcalin, à basses températures.

4. Procédé selon la revendication 3, caractérisé en ce que X représente l'hydrogène, le sodium ou le potassium.

5. Procédé selon la revendication 3 ou 4, caractérisé en ce qu'on utilise en tant que fluorure de métal alcalin le fluorure de sodium ou le fluorure de potassium, plus particulièrement le fluorure de sodium.

6. Procédé selon au moins l'une des revendications 3 à 5, caractérisé en ce que l'on utilise l'agent de fluoration, le fluor, en mélange avec des gaz inertes comme l'azote, SF₆, CF₄ ou des gaz nobles comme l'hélium, le néon, l'argon, le krypton, plus particulièrement l'azote.

7. Procédé selon au moins l'une des revendications 3 à 6 caractérisé en ce que l'on met en oeuvre la fluoration avec un mélange de N₂/F₂, qui a une teneur en F₂ comprise entre 1 et 15 % en volume, plus particulièrement entre 2 et 10 % en volume, de manière préférée entre 3 et 6 % en volume.

8. Procédé selon au moins l'une des revendications 3 à 7, caractérisé en ce que l'on utilise en tant que solvant des hydrocarbures halogénés, plus particulièrement le dichlorométhane, le chloroforme, le tétrachlorocarbone, le fluorotrichlorométhane, le trifluorotrichloréthane, le tétrafluorodichloréthane ou des nitriles, plus particulièrement l'acétonitrile.

9. Procédé selon au moins l'une des revendications 3 à 8, caractérisé en ce qu'on met en oeuvre la fluoration à une température de -80 à +10°C, plus particulièrement de -80 à -20°C, de manière préférée de -60 à -30°C, de manière particulièrement préférée de -50 à -35°C.

10. Utilisation des composés de formule (I) pour la fluoration de composés doués d'un caractère de carbanion évident ou occulté.

11. Utilisation selon la revendication 10, caractérisée en ce que l'on utilise des composés de 1,3-dicarbonyle en tant que composés à caractère de carbanion.
